# EUROPEAN PATENT SPECIFICATION

(11) **EP 3 155 968 B1**
(45) Date of publication and mention of the grant of the patent: **13.02.2019**
(21) Application number: 15382501.3
(22) Date of filing: 14.10.2015
(51) Int. Cl.: A61B 5/22, A61B 5/00, A61B 5/11, A63B 23/20

(54) **APPARATUS FOR MEASURING PELVIC FLOOR MUSCLE STRENGTH**
VORRICHTUNG ZUR MESSUNG DER MUSKELKRAFT DES BECKENBODENS
APPAREIL DE MESURE DE RÉSISTANCE DES MUSCLES DU PLANCHER PELVIEN

(43) Date of publication of application: 19.04.2017
(73) Proprietor: Oiarso, S. Coop., 20120 Hernani (ES)
(72) Inventor: ROZAS ELIZALDE, Pablo, 20120 HERNANI (ES); OTEGUI LAZKOZ, Susana, 20120 HERNANI (ES); ROMERO CULLERES, Georgia, 08243 MANRESA (ES); ARREGUI VICENTE, Miguel Angel, 20500 ARRASATE (ES); MARTINEZ RODRIGUEZ, Fernando, 20500 ARRASATE (ES); CAMPOS INSUNZA, Mikel Alberto, 20500 ARRASATE (ES)
(74) Representative: Igartua, Ismael

(56) References cited:
- EP-A1- 2 689 724
- RS-A- 20 090 196
- US-A- 4 971 036
- KATARINA PAREZANOVIC-ILIC ET AL: "THE ASSESSMENT OF KINESIS-THERAPEUTIC TREATMENT USING NUMERICAL EVALUATION OF PELVIC FLOOR MUSCLE FORCES", SERBIAN JOURNAL OF EXPERIMENTAL AND CLINICAL RESEARCH, vol. 10, no. 1, 1 January 2009 (2009-01-01), pages 23-26, XP055133110,
- KATARINA PAREZANOVIC-ILIC ET AL: "Muscle strength measurement of pelvic floor in women by vaginal dynamometer", SRPSKI ARHIV ZA CELOKUPNO LEKARSTVO, vol. 137, no. 9-10, 1 January 2009 (2009-01-01), pages 511-517, XP055133100, ISSN: 0370-8179, DOI: 10.2298/SARH0910511P

## Description

### TECHNICAL FIELD

The present invention relates to an apparatus for measuring pelvic floor muscle strength.

### PRIOR ART

The pelvic floor is a group of muscles and ligaments closing the abdominal cavity at the lower part thereof. Its function is to hold the pelvic organs in the suitable position because the normal working thereof depends on this.

The most common way of measuring pelvic floor muscle strength today is based on manual digital examination. Manual vaginal palpation, for quantification purposes, is based on the modified Oxford grading scale, with values of from 0 to 5, where 0 refers to the situation in which there is no muscle contraction and 5 refers to maximum muscle strength.

The use of manometers or perineometers is also known for measuring pelvic floor muscle strength. Manometry or perineometry should always be accurately measured in the same anatomical site and in the same conditions. There are a number of vaginal devices today for measuring pressures, all having different sizes and technical parameters, without being validated and with different pressure scales, i.e., mm of mercury or centimeters of water. This prevents the results obtained with different methods from being comparable.

In addition, patent document ES2397027A1 discloses a measuring device for measuring pelvic floor muscle strength comprising a speculum, a spring coupled to the grip area of the speculum and a reading module measuring the movement of the spring. This device calculates the contraction force in Newtons, calculating the difference between strength at rest and force obtained when performing the order to contract the pelvic floor muscles.

A similar measuring device is disclosed in patent document RS 20 090 196 A.

### DISCLOSURE OF THE INVENTION

The object of the invention is to provide an apparatus for measuring pelvic floor muscle strength, as described below.

The apparatus for measuring pelvic floor muscle strength of the invention comprises a speculum. Said speculum comprises a first part comprising a grip area and a front area, and a second part comprising a grip area and a front area, the second part being pivotally coupled to the first part.

The apparatus for measuring pelvic floor muscle strength further comprises a measuring device coupled to said speculum and suitable for obtaining the force applied on the front areas of the speculum when they are in contact with the vaginal wall. The measuring device comprises at least one elastic measuring element.

The measuring device comprises a rod which is fixed to the grip area of the first part of the speculum and traverses the grip area of the second part of the speculum. The measuring device further comprises pushing means coupled to the elastic measuring element.

The measuring device also comprises a coupling element for coupling and decoupling the pushing means and the rod, the coupling element being movable between a first position corresponding to a standby position of the apparatus wherein said pushing means are decoupled from the rod, and a second position corresponding to a measuring position of the apparatus wherein said pushing means are coupled to said rod.

The pushing means allow coupling the elastic measuring element to the rod once the apparatus is in the measuring position, such that the strength measurement is always taken from the same starting point, i.e., the state of the elastic measuring element at the beginning of the measurement is always the same, regardless of the opening of the front areas of the speculum. Calculation of the strength employed by the patient upon contracting the pelvic floor muscles is thereby simplified, because unlike the apparatus of the state of the art in which the elastic measuring element is always coupled to the grip areas of the speculum and strength of the elastic measuring element at the beginning of the measurement must therefore be stored, in this case, since the elastic measuring element always starts out in the same state, it is not necessary to measure the initial strength and subsequently subtract said initial strength from the obtained final strength.

These and other advantages and features of the invention will be come evident in view of the drawings and of the detailed description of the invention.

### DESCRIPTION OF THE DRAWINGS

Figure 1 shows a perspective view of the apparatus for measuring pelvic floor muscle strength according to one embodiment of the invention, the measuring device being shown in cross-section.
Figure 2 is a view of the apparatus of Figure 1.
Figure 3 is a view of the apparatus of Figure 1.
Figure 4 is a perspective view of the speculum of the apparatus of Figure 1.

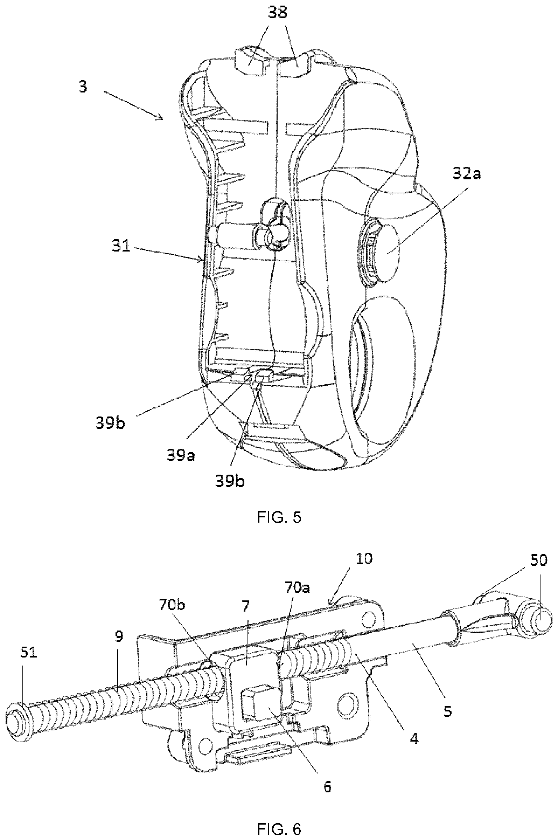

Figure 5 is a view of the measuring device of the apparatus of Figure 1.
Figure 6 is a view of part of the measuring device of the apparatus of Figure 1.
Figure 7 is a perspective view of the coupling element of the measuring device of the apparatus of Figure 1.
Figure 8 is a perspective view of the pusher of the measuring device of the apparatus of Figure 1.
Figure 9 is a perspective view of the first part of the receptacle of the measuring device of the apparatus of Figure 1.
Figure 10 is a perspective view of the receptacle with the coupling element of the measuring device of the apparatus of Figure 1.

### DETAILED DISCLOSURE OF THE INVENTION

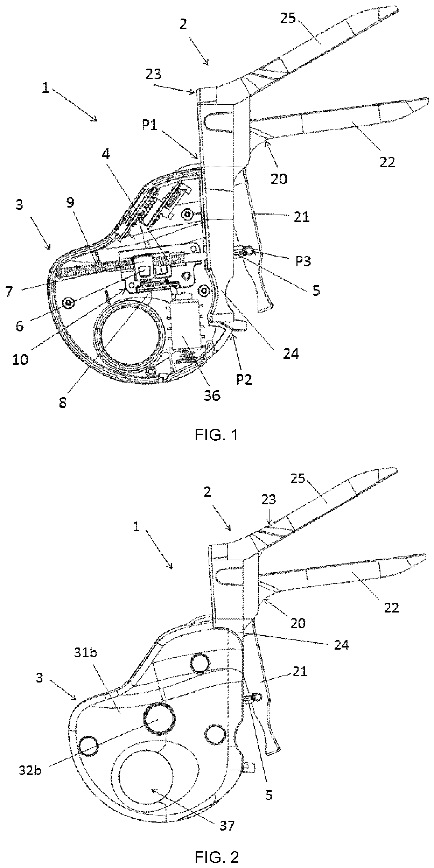

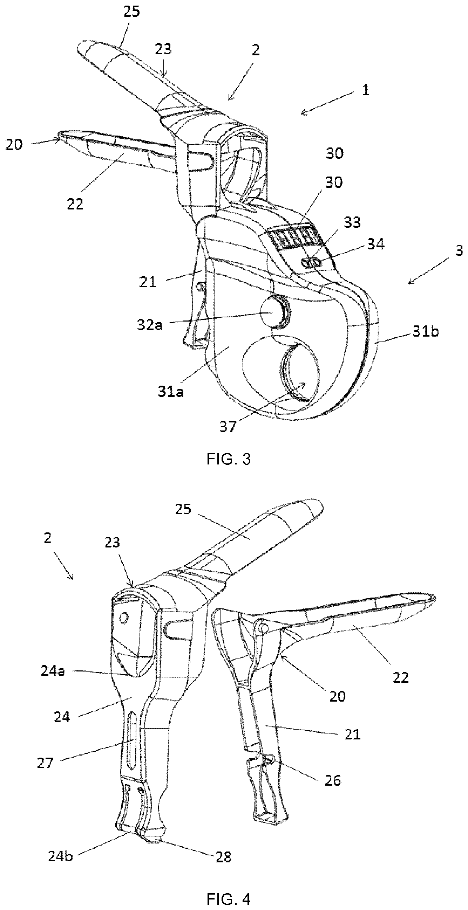

Figures 1 to 3 show an embodiment of the apparatus 1 for measuring pelvic floor muscle strength according to the invention.

The apparatus 1 for measuring pelvic floor muscle strength comprises a speculum 2 comprising a first part 20 comprising a grip area 21 and a front area 22, and a second part 23 comprising a grip area 24 and a front area 25, the second part 23 being pivotally coupled to the first part 20. The speculum is preferably made of a plastic material. The speculum is preferably disposable, so the speculum is used in a patient and disposed of after that use. Therefore, the speculum is replaced from one patient to the next, without having to sterilize it or use barrier means such as condoms, which can distort the strength measurement.

To understand this description, opening the speculum 2 will be considered the movement for separating the front parts 22 and 25 of said speculum 2. Opening the speculum 2 results in the grip areas 21 and 24 of the speculum 2 moving closer to one another. Likewise, closing the speculum 2 will be considered the movement for moving the front parts 22 and 25 of said speculum 2 closer to one another. Closing the speculum 2 results in the grip areas 21 and 24 of the speculum 2 being separated from one another.

The apparatus 1 for measuring pelvic floor muscle strength also comprises a measuring device 3 coupled to said speculum 2. The measuring device 3 is suitable for obtaining the force applied on the front areas 22 and 25 of the speculum 2 when they are in contact with the vaginal wall. In other words, once the front areas 22 and 25 of the speculum 2 are inserted into the vagina of a patient and it is assured that said front areas 22 and 25 are in contact with the vaginal wall, the measuring device 3 is suitable for measuring the force applied by the patient upon contracting the pelvic floor muscles. The measuring device 3 comprises at least one elastic measuring element 4.

The measuring device 3 also comprises a rod 5 which is fixed to the grip area 21 of the first part 20 of the speculum 2 and traverses the grip area 24 of the second part 23 of the speculum 2. In this embodiment the rod 5 comprises a cylindrical body preferably metallic.

The measuring device 3 further comprises pushing means coupled to the elastic measuring element 4, said pushing means being decoupled from the rod 5 when the apparatus 1 is in a standby position and the pushing means being coupled to said rod 5 when the apparatus 1 is in a measuring position.

To understand this description, it will be considered that the apparatus 1 is in a measuring position when the device is in the condition in which it can measure the force applied by a patient upon contracting the pelvic floor muscles. If the device is not in the condition of measuring said strength, it will be considered that the apparatus 1 is in the standby position.

In this embodiment the measuring device 3 comprises a coupling element 6 for coupling and decoupling the pushing means and the rod 5, said coupling element 6 being movable between a first position corresponding to the standby position of the apparatus 1 and a second position corresponding to the measuring position of the apparatus 1.

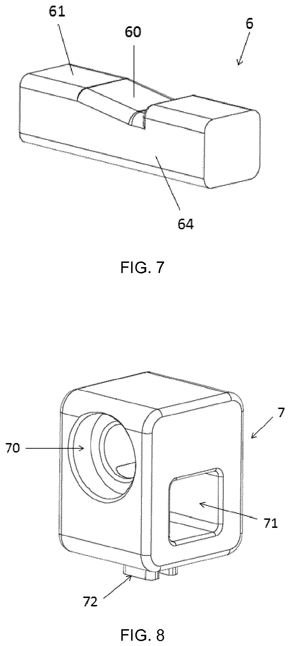

In this embodiment the pushing means comprise a pusher 7, shown in detail in Figure 8, cooperating with the elastic measuring element 4. The pusher 7 comprises a through hole 70 traversed by the rod 5. Therefore, when the coupling element 6 is in the first position the rod 5 can move with respect to the pusher 7, and when the coupling element 6 is in the second position the pusher 7 moves integrally with the rod 5.

In this embodiment the coupling element 6, shown in detail in Figure 7, is an axially movable part which presses the rod 5 against the pusher 7 in the second position, i.e., when the apparatus 1 is in the measuring position.

In this embodiment the pusher 7 comprises a second through hole 71 communicated with the hole 70 traversed by the rod 5, the coupling element 6 being housed in said second hole 71. The second through hole 71 is perpendicular to the hole 70 traversed by the rod 5.

The coupling element 6 of this embodiment comprises a body 64 comprising a first section 60 and a second section 61, said first section 60 having a smaller size than said second section 61. When the coupling element 6 is in the first position, the first section 60 is arranged at the same level as the rod 5 and there is no contact between said first section 60 and the rod 5, such that the rod 5 can move with respect to the pusher 7. In contrast, when the coupling element 6 is in the second position, the second section 61 is arranged at the same level as the rod 5 and said second section 61 has a dimension such that it presses the rod 5 against the pusher 7. Therefore due to static friction force, the pusher 7 and the coupling element 6 move integrally with the rod 5.

In this embodiment the elastic measuring element 4 is a coil spring that is coaxial with the rod 5 which is supported on the pushing means. The choice of the elastic constant of the measuring spring is based on the range of forces to be measured.

In this embodiment the measuring device 3 comprises a potentiometer 8 coupled to the pushing means. The potentiometer 8 is preferably a linear potentiometer comprising a sliding cursor, the pushing means preferably comprising a clamp 72 coupled to said cursor.

As is known for the person skilled in the art, a potentiometer comprises three terminals. The resistor between the side terminals is fixed, whereas the resistor between each side terminal and the central terminal varies according to the position of the sliding cursor of the potentiometer. As described above, when the coupling element 6 is in the second position, the rod 5 and the pushing means move in an integral manner. Therefore, when the coupling element 6 is in the second position, the movement for closing the front areas 22 and 25 of the speculum 2 causes the movement of the rod 5, and accordingly of the pushing means, which in turn are coupled to the sliding cursor of the potentiometer 8, the resistor of the potentiometer 8 changing between the side terminals and the central terminal thereof. By knowing the elastic constant of the measuring spring and by knowing how much the measuring spring is compressed as a result of the potentiometer resistor variation, the force applied by the patient can be calculated.

In this embodiment the measuring device 3 comprises an elastic opening element 9 coupled to the rod 5 and suitable for opening the front areas 22 and 25 of the speculum 2 until they are in contact with the vaginal wall when the apparatus 1 is in the standby position. In other possible embodiments in which the measuring device does not comprise an elastic opening element, the physician will have to separate the front areas of the speculum 2 until they are in contact with the vaginal wall.

The elastic opening element 9 of this embodiment is in the natural state, i.e., at rest, when the front areas 22 and 25 of the speculum 2 are open. When the physician inserts the front areas 22 and 25 of the speculum 2 into the vagina, said physician must first close said front areas 22 and 25. When the front areas 22 and 25 are closed, the elastic opening element 9 is compressed. Once the front areas 22 and 25 are inserted into the vagina, when the physician no longer applies force on grip areas 21 and 24 to keep the speculum 2 closed, the elastic opening element 9 tries to return to its natural state, pushing the rod 5 and therefore the grip area 21 of the first part 20 of the speculum 2 until the front areas 22 and 25 of the speculum 2 touch the vaginal wall.

The elastic opening element 9 enables the front areas 22 and 25 of the speculum 2 to be in contact with the vaginal wall such that when switching the apparatus 1 to the measuring position and the voluntary contraction of the patient taking place, the measuring device 3 can correctly record the force applied by the patient.

The position for opening the front areas 22 and 25 of the speculum 2 varies from patient to patient. When the apparatus 1 is switched to the measurement position, the measuring device 3 measures the contraction force without distinction of the starting point or the opening of said front areas 22 and 25 of the speculum 2.

The function of the elastic opening element 9 is to enable the blades to be in contact with the vaginal wall before switching the apparatus 1 to the measuring position and to remain that way while switching the apparatus 1 from a standby position to a measuring position. Therefore when the patient contracts the pelvic floor muscles, the measuring device 3 records the entire contraction capacity of the pelvic muscle, because if there had been a free space between the vaginal wall and the front areas 22 and 25 of the speculum 2, the measurement would not be correct.

In this embodiment the measuring device 3 comprises stop means preventing the elastic opening element 9 from opening the front areas 22 and 25 of the speculum 2 when the apparatus 1 switches to the measuring position. Said stop means cooperate with the pushing means.

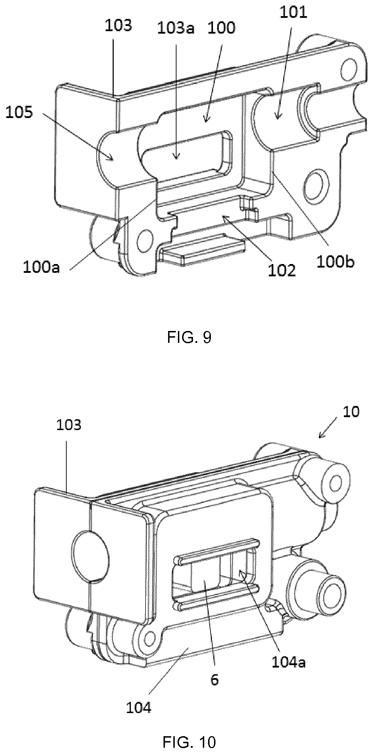

In this embodiment the measuring device 3 comprises a receptacle 10, shown in detail in Figures 9 and 10. The receptacle 10 comprises a first housing 100 in which the pusher 7 is arranged. The first side wall 100a and the second side wall 100b of said first housing 100 demarcate the path of the pusher 7. The first side wall abuts with the pusher 7 when the apparatus 1 switches to the measuring position. As explained above, when the apparatus 1 switches to the measuring position, i.e., when the coupling element 6 moves to the second position, the pusher 7 is coupled to the rod 5 and therefore the pusher 7 moves integrally with said rod 5. Although in this measuring position the elastic opening element 9 is compressed and wants to return to its natural state, the first side wall 100a of the first housing 100 of the receptacle 10 prevents movement of the pusher 7, because the pusher 7 is at the boundary of its path in this sense. Since the pusher 7 and the rod 5 are coupled to one another, movement of the rod 5 so as to separate the front areas 22 and 25 of the speculum 2 from one another is not possible. When the coupling element 6 is in the second position, i.e., when the apparatus 1 is in the measuring position, the front areas 22 and 25 of the speculum 2 only can be moved so as to close.

When the coupling element 6 is in the first position, the pusher 7 therefore being decoupled from the rod 5, the pusher 7 is arranged in contact with the first side wall 100a of the first housing 100 of the receptacle 10, because the elastic measuring element 4 is compressed and applies a force on the surface 70a of the pusher 7.

In this embodiment the elastic opening element 9 is a coil spring that is coaxial with the rod 5 which is supported on the pushing means. The elastic opening element 9 is arranged between the stop 51 comprised in the rod 5 at one of its ends and the surface 70b of the pusher 7. Therefore, when the apparatus 1 is in the standby position, the pusher 7 therefore being decoupled from the rod 5, if the elastic opening element 9 is compressed it applies a force on the rod 5, moving it until the elastic opening element 9 is at rest. In this embodiment the elastic constant of the measuring spring 4 is greater than the elastic constant of the opening spring 9, so since the pusher 7 is arranged between both springs, even though the elastic opening element 9 is compressed it does not have enough strength to move the pusher 7 because the force applied by the measuring spring 4 on said pusher 7 is always greater than the force applied by the opening spring 9 on the pusher 7.

The elastic constant of the opening spring 9 is preferably low because a spring with a high elastic constant could hurt the patient, applying high pressure on the vaginal wall.

The receptacle 10 of this embodiment comprises a second housing 101 communicated with the first housing 100, the elastic measuring element 4 being arranged in said first housing 100 and said second housing 101. The receptacle 10 further comprises a third housing 102 communicated with the first housing 100, the potentiometer 8 being arranged in said third housing 102. The receptacle 10 comprises a fourth housing 105 communicated with the first housing 100, the elastic opening element 9 being partially housed in said fourth housing 105. The rod 5 traverses the receptacle 10, part of said rod 5 being housed in the first, second and fourth housings 100, 101 and 105 of the receptacle 10.

In this embodiment the receptacle 10 is formed by a first part 103 and a second part 104 which are fixed to one another. The first and second parts 103 and 104 of the receptacle 10 comprise a respective opening 103a and 104a arranged facing one another. The body 64 of the coupling element 6 is arranged inside the receptacle 10 between said openings 103a and 104a.

In this embodiment the measuring device 3 comprises a casing 31 fixed to the grip area 24 of the second part 23 of the speculum 2, the pushing means and part of the rod 5 being housed in said casing 31. The casing 31 and the rod 5 are attached to the speculum 2 in a detachable manner, the casing 31 being fixed to the grip area 2 of the second part 23 through anchoring means.

In this embodiment the measuring device 3 is fixed to the speculum 2 in a quick and easy manner. The measuring device 3 and the speculum 2 are fixed to one another by means of three attachment points P1, P2 and P3. On one hand the casing 31 is fixed to the grip area 24 of the second part 23 of the speculum 2 at points P1 and P2. Furthermore, the rod 5 is fixed to the grip area 21 of the first part 20 of the speculum 2 at point P3.

In this embodiment the casing 31 comprises a housing suitable for receiving at least one part of the grip area 24 of the second part 23 of the speculum 2. Said housing comprises at least one flange 38 in the upper part of the housing and at least one groove 39a and at least one stop 39b in the lower part of the housing.

In this embodiment the grip area 21 of the first part 20 of the speculum 2 comprises a housing 26 in which one end of the rod 5 is housed. To that end, the end of the rod 5 comprises two projections 50 which are housed in said housing 26.

In this embodiment the grip area 24 of the second part 23 of the speculum 2 comprises an opening 27 suitable for allowing the end of the rod 5 that is going to be fixed to the first part 20 to traverse the grip area 24 of the second part 23. The grip area 24 of the second part 23 further comprises a projection 28 suitable for being housed in the groove 39a of the casing 31.

In this embodiment the speculum 2 is fixed to the measuring device 3 as described below. First of all, the end of the rod 5 is passed through the opening 37 of the grip area 24 of the second part 23. Then the upper surface 24a of the grip area 24 of the second part 23 is inserted in the flanges 38 of the casing 31, forming attachment point P1. The lower surface 24b of the grip area 24 is then positioned such that the projection 28 of the grip area 24 is housed in the groove 39a of the casing 31 and the stops 39b of the casing 31 retain said lower surface 24b of the grip area 24, forming attachment point P2. Finally, the projections 50 of the rod 5 are inserted in the housing 26 of the grip area 21 of the first part 20, forming attachment point P3.

In this embodiment the casing 31 comprises a first half 31a and a second half 31b which are fixed to one another, preferably by means of screws. The first half 31a of the casing 31 comprises an opening in which a measurement button 32a cooperating with the coupling element 6 is arranged, such that by pressing the measurement button 32a the apparatus 1 will be in the measuring position and the coupling element 6 is positioned in the second position. The second half 31b comprises an opening in which a standby button 32b cooperating with the coupling element 6 is arranged, such that by pressing the standby button 32b the apparatus 1 will be in the measuring position and the coupling element 6 is positioned in the first position.

In this embodiment the casing 31 comprises a hole 37 that makes it easier to handle the apparatus 1. The physician can insert his or her thumb through this hole 37 such that it allows the physician to hold the apparatus 1 more comfortably.

In this embodiment the measuring device 3 comprises a display 30 suitable for showing the force applied on the front areas 22 and 25 of the speculum 2. The casing 31 comprises a rectangular opening in which said display 30 is arranged. The measuring device 3 also comprises an on button 33 and a reset button 34.

In this embodiment the measuring device 3 comprises a circuit board comprising a microprocessor in which the force applied by the patient is calculated according to the variation of the resistor in the potentiometer 8.

In this embodiment the measuring device 3 comprises a power source 36 for being able to power the device electronics; preferably a battery or cell is used. In other possible embodiments any other type of power source known by the person skilled in the art can be used.

Measuring pelvic floor strength with the apparatus 1 of this embodiment can be done as follows:
- With the apparatus 1 in the standby position, i.e., the coupling element 6 being in the first position, the physician brings the front areas 22 and 25 of the speculum 2 together and inserts them into the vagina of the patient.
- Once inside the vagina, the front areas 22 and 25 are separated until they are in contact with the vaginal wall. In this embodiment the separation of the front areas 22 and 25 is done by the elastic opening element 9.
- The coupling element 6 then moves to the second position, arranging the apparatus 1 in the measuring position.
- Once the coupling element 6 is in the measuring position the patient is told to contract pelvic floor muscles.
- Once the force made is measured, the coupling element 6 moves to the first position to arrange the apparatus 1 in the standby position.
- The physician brings the front areas 22 and 25 of the speculum 2 together and removes them.

## Claims

1. Apparatus for measuring pelvic floor muscle strength comprising a speculum (2) comprising a first part (20) comprising a grip area (21) and a front area (22), and a second part (23) comprising a grip area (24) and a front area (25), the second part (23) being pivotally coupled to the first part (20), and a measuring device (3) coupled to said speculum (2) suitable for obtaining the force applied on said front areas (22, 23) when they are in contact with the vaginal wall, said measuring device (3) comprising at least one elastic measuring element (4), the measuring device (3) comprising a rod (5) which is fixed to the grip area (21) of the first part (20) of the speculum (2) and traverses the grip area (24) of the second part (23) of the speculum (2), and pushing means coupled to the elastic measuring element (4), **characterized in that** the measuring device (3) comprises a coupling element (6) for coupling and decoupling the pushing means and the rod (5), said coupling element (6) being movable between a first position corresponding to a standby position of the apparatus (1) wherein said pushing means are decoupled from the rod (5), and a second position corresponding to a measuring position of the apparatus (1) wherein said pushing means are coupled to said rod (5).

2. Apparatus according to claim 1, wherein the pushing means comprise a pusher (7) cooperating with the elastic measuring element (4), said pusher (7) comprising a through hole (70) traversed by the rod (5), such that when the coupling element (6) is in the first position the rod (5) can move with respect to the pusher (7), and when the coupling element (6) is in the second position the pusher (7) moves integrally with the rod (5).

3. Apparatus according to claim 2, wherein the coupling element (6) is an axially movable part which presses the rod (5) against the pusher (7) in the second position.

4. Apparatus according to claim 3, wherein the pusher (7) comprises a second through hole (71) communicated with the hole (70) traversed by the rod (5), being the coupling element (6) housed in said second hole (71).

5. Apparatus according to claim 4, wherein the second through hole (71) is perpendicular to the hole (70) traversed by the rod (5).

6. Apparatus according to any of the preceding claims, wherein the elastic measuring element (4) is a coil spring that is coaxial with the rod (5) which is supported on the pushing means.

7. Apparatus according to any of the preceding claims, wherein the measuring device (3) comprises a potentiometer (8) coupled to the pushing means, the potentiometer (8) preferably comprising a sliding cursor and preferably comprising the pushing means a clamp (72) coupled to said cursor.

8. Apparatus according to any of the preceding claims, wherein the measuring device (3) comprises an elastic opening element (9) coupled to the rod (5) and suitable for opening the front areas (22, 25) of the speculum (2) until they are in contact with the vaginal wall when the apparatus (1) is in the standby position.

9. Apparatus according to the preceding claim, comprising stop means preventing the elastic opening element (9) from opening the front areas (22, 25) of the speculum (2) when the apparatus (1) switches to the measuring position.

10. Apparatus according to the preceding claim, wherein said stop means cooperate with the pushing means.

11. Apparatus according to claim 9 or 10, wherein the measuring device (3) comprises a receptacle (10) in which the pushing means are housed, the receptacle (10) comprising stop means.

12. Apparatus according to any of claims 8 a 11, wherein the elastic opening element (9) is a coil spring that is coaxial with the rod (5) which is supported on the pushing means.

13. Apparatus according to any of the preceding claims, wherein the measuring device (3) comprises a casing (31) fixed to the grip area (24) of the second part (23) of the speculum (2), the pushing means and part of the rod (5) being housed in said casing (31).

14. Apparatus according to claim 13, wherein the casing (31) and the rod (15) are attached to the speculum (2) in a detachable manner, the casing (31) being fixed to the grip area (24) of the second part (23) through anchoring means.

## Patentansprüche

1. Vorrichtung zum Messen der Muskelkraft des Beckenbodens, umfassend ein Spekulum (2) mit einem ersten Teil (20) mit einem Griffbereich (21) und einem vorderen Bereich (22) sowie einem zweiten Teil (23) mit einem Griffbereich (24) und einem vorderen Bereich (25), wobei der zweite Teil (23) schwenkbar mit dem ersten Teil (20) gekoppelt ist, und eine Messvorrichtung (3), die mit dem Spekulum (2) gekoppelt ist, und geeignet ist, um die auf die vorderen Bereiche (22, 23) ausgeübte Kraft zu erhalten, wenn diese in Kontakt mit der Vaginalwand stehen, wobei die Messvorrichtung (3) mindestens ein elastisches Messelement (4) umfasst, wobei die Messvorrichtung (3) eine Stange (5) umfasst, die am Griffbereich (21) des ersten Teils (20) des Spekulums (2) befestigt ist, und den Griffbereich (24) des zweiten Teils (23) des Spekulums (2) durchquert, und Schubeinrichtungen, die mit dem elastischen Messelement (4) verbunden sind, **dadurch gekennzeichnet, dass** die Messvorrichtung (3) ein Koppelelement (6) zum Koppeln und Entkoppeln der Schubeinrichtungen und der Stange (5) umfasst, wobei das Koppelelement (6) zwischen einer ersten Position, die einer Bereitschaftsposition der Vorrichtung (1) entspricht, in der die Schubeinrichtungen von der Stange (5) entkoppelt sind, und einer zweiten Position entspricht, die einer Messposition der Vorrichtung (1) entspricht, in der die Schubeinrichtungen an die Stange (5) gekoppelt sind.

2. Vorrichtung nach Anspruch 1, wobei die Schubeinrichtungen einen Schieber (7) umfassen, der mit dem elastischen Messelement (4) zusammenwirkt, wobei der Schieber (7) ein Durchgangsloch (70) aufweist, das von der Stange (5) durchquert wird, sodass sich das Koppelelement (6) in der ersten Position befindet, in der sich die Stange (5) relativ zum Schieber (7) bewegen kann, und wenn sich das Koppelelement (6) in der zweiten Position befindet, sich der Schieber (7) integral mit der Stange (5) bewegt.

3. Vorrichtung nach Anspruch 2, wobei das Koppelelement (6) ein axial beweglicher Teil ist, der die Stange (5) in der zweiten Position gegen den Schieber (7) schiebt.

4. Vorrichtung nach Anspruch 3, wobei der Schieber (7) ein zweites Durchgangsloch (71) umfasst, das mit dem von der Stange (5) durchquerten Loch (70) in Verbindung steht, wobei es sich um das in dem zweiten Loch (71) untergebrachte Koppelelement (6) handelt.

5. Vorrichtung nach Anspruch 4, wobei das zweite Durchgangsloch (71) senkrecht zu dem von der Stange (5) durchquerten Loch (70) ist.

6. Vorrichtung nach einem der vorhergehenden Ansprüche, wobei das elastische Messelement (4) eine Schraubenfeder ist, die koaxial zu der Stange (5) ist, die auf den Schubeinrichtungen abgestützt wird.

7. Vorrichtung nach einem der vorhergehenden Ansprüche, wobei die Messvorrichtung (3) ein Potenziometer (8) umfasst, das mit den Schubeinrichtungen gekoppelt ist, wobei das Potenziometer (8) vorzugsweise einen gleitenden Zeiger umfasst und vorzugsweise die Schubeinrichtungen eine an den Zeiger gekoppelte Klammer (72) umfassen.

8. Vorrichtung nach einem der vorhergehenden Ansprüche, wobei die Messvorrichtung (3) ein elastisches Öffnungselement (9) umfasst, das an die Stange (5) gekoppelt, und zum Öffnen der vorderen Bereiche (22, 25) des Spekulums (2) geeignet ist, bis sie in Kontakt mit der Vaginalwand stehen, wenn sich die Vorrichtung (1) in der Bereitschaftsposition befindet.

9. Vorrichtung nach dem vorhergehenden Anspruch, umfassend eine Anschlagseinrichtung, die verhindert, dass das elastische Öffnungselement (9) die vorderen Bereiche (22, 25) des Spekulums (2) öffnet, wenn die Vorrichtung (1) in die Messposition wechselt.

10. Vorrichtung nach dem vorhergehenden Anspruch, wobei die Anschlagseinrichtungen mit den Schubeinrichtungen zusammenwirken.

11. Vorrichtung nach Anspruch 9 oder 10, wobei die Messvorrichtung (3) eine Aufnahme (10) umfasst, in der die Schubeinrichtungen untergebracht sind, wobei die Aufnahme (10) Anschlagseinrichtungen umfasst.

12. Vorrichtung nach einem der Ansprüche 8 bis 11, wobei das elastische Öffnungselement (9) eine Schraubenfeder ist, die koaxial zu der Stange (5) ist, die auf den Schubeinrichtungen abgestützt wird.

13. Vorrichtung nach einem der vorhergehenden Ansprüche, wobei die Messvorrichtung (3) ein Gehäuse (31) umfasst, das an dem Griffbereich (24) des zweiten Teils (23) des Spekulums (2) der Schubeinrichtungen und eines Teils davon befestigt ist, wobei die Stange (5) in dem Gehäuse (31) untergebracht ist.

14. Vorrichtung nach Anspruch 13, wobei das Gehäuse (31) und die Stange (15) abnehmbar an dem Spekulum (2) befestigt sind, wobei das Gehäuse (31) an dem Griffbereich (24) des zweiten Teils (23) durch Verankerungsmittel befestigt ist.

## Revendications

1. Appareil de mesure de résistance des muscles du plancher pelvien comprenant un spéculum (2) comprenant une première partie (20) comprenant une zone de préhension (21) et une zone avant (22), ainsi qu'une seconde partie (23) comprenant une zone de préhension (24) et une zone avant (25), la seconde partie (23) étant accouplée de manière pivotante à la première partie (20), ainsi qu'un dispositif de mesure (3) accouplé audit spéculum (2) permettant d'obtenir la force appliquée sur lesdites zones avant (22, 23) lorsqu'elles sont en contact avec la paroi vaginale, ledit dispositif de mesure (3) comprenant au moins un élément de mesure élastique (4), le dispositif de mesure (3) comprenant une tige (5) qui est fixée à la zone de préhension (21) de la première partie (20) du spéculum (2) et traverse la zone de préhension (24) de la seconde partie (23) du spéculum (2), ainsi que des moyens de poussée accouplés à l'élément de mesure élastique (4), **caractérisé en ce que** le dispositif de mesure (3) comprend un élément d'accouplement (6) pour accoupler et découpler les moyens de poussée et la tige (5), ledit élément d'accouplement (6) étant mobile entre une première position correspondant à une position d'attente de l'appareil (1) dans laquelle lesdits moyens de poussée sont découplés de la tige (5) et une seconde position correspondant à une position de mesure de l'appareil (1) dans laquelle lesdits moyens de poussée sont accouplés à ladite tige (5).

2. Appareil selon la revendication 1, dans lequel les moyens de poussée comprennent un poussoir (7) coopérant avec l'élément de mesure élastique (4), ledit poussoir (7) comprenant un trou traversant (70) traversé par la tige (5), de sorte que, lorsque l'élément d'accouplement (6) est dans la première position, la tige (5) puisse se déplacer par rapport au poussoir (7) et que, lorsque l'élément d'accouplement (6) est dans la seconde position, le poussoir (7) se déplace d'un seul tenant avec la tige (5).

3. Appareil selon la revendication 2, dans lequel l'élément d'accouplement (6) est une partie mobile axialement qui presse la tige (5) contre le poussoir (7) dans la seconde position.

4. Appareil selon la revendication 3, dans lequel le poussoir (7) comprend un second trou traversant (71) en communication avec le trou (70) traversé par la tige (5), constituant l'élément d'accouplement (6) logé dans ledit second trou (71).

5. Appareil selon la revendication 4, dans lequel le second trou traversant (71) est perpendiculaire au trou (70) traversé par la tige (5).

6. Appareil selon l'une quelconque des revendications précédentes, dans lequel l'élément de mesure élastique (4) est un ressort hélicoïdal coaxial à la tige (5) qui est supportée par les moyens de poussée.

7. Appareil selon l'une quelconque des revendications précédentes, dans lequel le dispositif de mesure (3) comprend un potentiomètre (8) accouplé aux moyens de poussée, le potentiomètre (8) comprenant de préférence un curseur coulissant et comprenant de préférence dans les moyens de poussée une pince (72) accouplée audit curseur.

8. Appareil selon l'une quelconque des revendications précédentes, dans lequel le dispositif de mesure (3) comprend un élément d'ouverture élastique (9) accouplé à la tige (5) et adapté pour ouvrir les zones avant (22, 25) du spéculum (2) jusqu'à ce qu'elles entrent en contact avec la paroi vaginale lorsque l'appareil (1) est en position d'attente.

9. Appareil selon la revendication précédente, comprenant des moyens d'arrêt empêchant l'élément d'ouverture élastique (9) d'ouvrir les zones avant (22, 25) du spéculum (2) lorsque l'appareil (1) bascule en position de mesure.

10. Appareil selon la revendication précédente, dans lequel lesdits moyens d'arrêt coopèrent avec les moyens de poussée.

11. Appareil selon la revendication 9 ou 10, dans lequel le dispositif de mesure (3) comprend un réceptacle (10) dans lequel sont logés les moyens de poussée, le réceptacle (10) comprenant des moyens d'arrêt.

12. Appareil selon l'une quelconque des revendications 8 à 11, dans lequel l'élément d'ouverture élastique (9) est un ressort hélicoïdal coaxial à la tige (5) qui est supportée par les moyens de poussée.

13. Appareil selon l'une quelconque des revendications précédentes, dans lequel le dispositif de mesure (3) comprend un boîtier (31) fixé à la zone de préhension (24) de la seconde partie (23) du spéculum (2), les moyens de poussée et une partie de la tige (5) étant logés dans ledit boîtier (31).

14. Appareil selon la revendication 13, dans lequel le boîtier (31) et la tige (15) sont fixés au spéculum (2) de manière détachable, le boîtier (31) étant fixé à la zone de préhension (24) de la seconde partie (23) par des moyens d'ancrage.
